# EUROPÄISCHE PATENTSCHRIFT

(11) **EP 1 077 073 B1**
(45) Veröffentlichungstag und Bekanntmachung des Hinweises auf die Patenterteilung: **13.10.2004**
(21) Anmeldenummer: 99116119.1
(22) Anmeldetag: 18.08.1999
(51) Int. Cl.: A61L 31/00, C08G 63/08, A61K 7/40

(54) **Resorbierbare Copolylactide und ihre Verwendung**
Resorbable copolylactides and their use
Copolylactides résorbables et leur utilisation

(43) Veröffentlichungstag der Anmeldung: 21.02.2001
(73) Patentinhaber: Jürgens, Christian, Dr. med., 20149 Hamburg (DE)
(72) Erfinder: Jürgens, Christian, Dr. med., 20149 Hamburg (DE); Kricheldorf, Hans R., Prof. Dr., 22607 Hamburg (DE); Kreise-Saunders, Ingrid, Dr., 28195 Bremen (DE)
(74) Vertreter: Benz, Jürgen

(56) Entgegenhaltungen:
- EP-A- 0 509 203
- EP-A- 0 768 329
- EP-A- 0 782 865
- US-A- 4 045 418

## Beschreibung

Die Erfindung betrifft neue Copolylactide, welche aus Einheiten von racemischen Lactid und den Comonomeren ε- Caprolacton, δ- Valerolacton, 1,4-Dioxanon-2 oder 1,3-Dioxanon-2 in einem Molverhältnis Lactid/Comonomer von 90-80 / 10-20 in Gegenwart von Zinn(II)-diethylhexanoat als Initiator und eines Cokatalysators bei etwa 160°C polymerisiert wurden, und eine Glastemperatur zwischen 30 und 43°C, ein Molekulargewicht Mₙ von 15.000 bis 50.000 und eine Polydispersität Pₙ (M_{w} / Mₙ) zwischen 1,2 und 2 aufweisen. Die neuen Copolylactide eigenen sich insbesondere in hervorragender Weise bei der Versorgung von Wunden aller Art.

Die US 4,045,418 beschreibt ein Copolymer bestehend aus D,L-Lactid und ε-Caprolacton in Gegenwart von Zinn (II)-caprylat als Initiator. Das Copolymer ist transparent, rigide und brüchig und findet als Wegwerfprodukt und Formteil in der Automobilindustrie, im Haushalt und der Verpackungsindustrie Verwendung. Die Molekulargewichte liegen zwischen 100.000 und 300.000. Für medizinische Anwendungen z.B. als Operationsinzisionsfolie oder als flüssiger Handschuh kommen diese Copolymere in gelöster Form nicht in Betracht, da sie nach dem Aufsprühen auf die Haut unter Verdunstung des Lösungsmittel als Film aushärten und daher brüchig und rissig werden.

Aus der FR-2126270 sind filmbildende Polymere aus Milchsäure und Glykolsäure in Lösungsmitteln wie z.B. Ethylacetat bekannt, die zusätzlich noch pharmakologische Wirkstoffe enthalten und beispielsweise als Sprühverband eingesetzt werden können.

Nachteilig an diesen Polylactonen ist die Tatsache, daß sie nur als Wirkstoffträger auf intakter Haut in Frage kommen, da das Polymer/Drug-Gemisch stets aus Lösung auf die Haut aufgebracht wird. Die verwendetenLösungsmittel Chloroform, Difluordichlormethan oder Ethylacetat würden jedoch eine offene Wunde intensiv schädigen.

Die EP-B1-270 987 beschreibt ein Verfahren zur Herstellung katalysatorfreier resorbierbarer Homopolymere oder Copolymere auf der Basis von Hydroxysäuren. Es werden hauptsächlich Lactid und Glycolid zur Copolymerbildung eingesetzt. Glycolid ist jedoch ungeeignet, um Copolymerisate für die angestrebten topischen Anwendungen zu erhalten, weil die Copolymere des Glycolids zu schnell abbauen.

EP-0 768 329 beschreibt ein Blockcopolymer bestehend aus Lactide und 1,4-Dioxanon-2, das als Film in der Adhäsionsprophylate und als hemostatischer Schaum verwendet wird.

Gegenstand der EP-0 509 203 ist die Verwendung von Copolymeren aus racemischen Lactid und ε-Caprolacton, δ-Valerolacton, γ-Decalacton oder β-Hydroxybuttersäure, hergestellt durch die Umsetzung der Monomere im Molverhältnis Lactid zu Reaktionspartner von etwa 95 bis 70 zu 5 bis 30 unter Zugabe von Zinn (II)-di(ethylhexanoat) als Initiator bei Temperaturen von etwa 150 °C über eine Zeitspanne von 16 bis 48 Stunden, für die topische Behandlung menschlicher oder tierischer Haut. Das genannte ältere Schutzrecht der Anmelderin schildert dann weiterhin, daß für topische Anwendungen am geeignetsten Copolymere sind, die ein Molverhältnis Initiator zu Reaktionspartner von etwa 1 : 100 bis 1 : 500 besitzen. Zur Entfernung von verbleibenden Monomeren, kurzkettigen Oligomeren oder gegebenenfalls auch überschüssigen Weichmachern wird die Reaktionsmasse in der Regel mit der 600- 800 fachen Menge Alkohol ausgefällt.
Die gemäß der Lehre der EP 0509203 erzielten Coplymere zeigen zwar gegenüber den Polymerprodukten der US 4,045,418 Eigenschaften, die geeigneter für die topische Wundbehandlung sind. Sie weisen aber noch immer nicht optimale Merkmale in Bezug auf Flexibilität, Haftbarkeit, Vermeidung von Klebrigkeit etc. auf. Überdies erhält man oft, folgt man den offenbarten Herstellungsbedingungen, Produkte mit nicht reproduzierbaren und gelegentlich nicht brauchbaren Eigenschaften.

Die Lehre der vorliegenden Erfindung geht von der Aufgabe aus, resorbierbare, physiologisch unbedenkliche Copolylactide der geschilderten Art in mehrfacher Weise weiterhin zu verbessern.
Wichtig dabei ist die Tatsache, daß die Anwendung auf unverletzter Haut Copolylactide mit hoher Adhärenz, Flexibilität und Dehnbarkeit erfordert. Wasserdampfpermeabilität ist hier von geringerer Bedeutung als bei der Anwendung auf Wunden, und der Abbau kann auch etwas schneller erfolgen, sollte aber auch im wässrigen Milieu zumindest 24 Stunden bei einer Schichtdicke von ca. 3-5 µm nicht unterschreiten. Undurchlässigkeit für hydrophile und lipophile Allergene ist zu verlangen. Daher sind auch für diese Anwendungen amorphe Copolylactide, allerdings mit niedriger Viskosität (=kurzkettige Polymere) zu bevorzugen.
Für die Verwendung auf verletzter Haut ist die Wasserdampfpermeabilität ein entscheidendes Kriterium und kann nicht allein durch die Copolymere erreicht werden, da diese hydrophob und wenig durchlässig sind (< 60 ml/h/m²). Hier bedarf es entweder eines relativ hohen Monomeranteils im Reaktionsprodukt oder aber des Zusatzes hygroskopischer/hydrophiler Substanzen (z.B. Glycerin), durch die eine Dampfdurchlässigkeit von initial hohen Werten (> 150 ml/h/m²) erreicht wird, die im Verlauf von Tagen abnimmt (entsprechend der Wundsekretion).

Die Undurchlässigkeit für Keime ist bei einer Schichtdicke von 25- 50 pm für einen Zeitraum von mindestens 14 Tagen zu verlangen. Dieses läßt sich nur durch längere Molekülketten (= höhere Viskosität) erreichen.

Ferner ist es wichtig, daß die für den vorbestimmten Verwendungszweck vorgesehenen Copolymere keine klebrige Konsistenz aufweisen.
Diese Aufgabe wird nun durch die erfindungsgemäßen Copolylactide gelöst.

Gegenstand der Erfindung ist somit ein flexibles, transparentes Copolylactid, welches aus polymerisierten Einheiten von racemischen Lactid und den Comonomeren ε- Caprolacton, δ- Valerolacton, 1,4-Dioxanon-2 oder 1,3-Dioxanon-2 aufgebaut ist, eine Glasübergangstemperatur (Tg) zwischen 30 und 43°C, ein Zahlenmittel des Molekulargewichts Mₙ von 15.000 bis 50.000 und eine Molekulargewichtsverteilung (Polydispersität Pₙ ) von 1,2 bis 2 aufweist. Daraus ergibt sich, daß das Molekulargewicht M_{w} des Copolylactids einen Wert von deutlich unter 100.000 einnimmt.

Die Eigenschaften dieser erfindungsgemäßen Copolylactide unterscheiden sich deutlich von den in der US 4,045,418 und der EP 0509 203, wie aus der unten stehenden Tabelle zu entnehmen ist und weiter unten erläutert wird. So liegt, wie in Vergleichsbeispielen gezeigt wurde, beispielsweise die Glastemperatur der Copolymere der EP 0509 203 deutlich unter 30°C, während sie bei den Produkten der US 4,045,418 über 45°C liegt.

Besonders vorteilhaft und bevorzugt im Sinne der Erfindung sind Copolylactide, die eine Glasübergangstemperatur von 30 bis 40°C, insbesondere 33 bis 37°C aufweisen und ein Zahlenmittel des Molekulargewichts Mₙ von 25.000 bis 40.000 besitzen bei einer Polydispersität von 1,4 bis 1,7. Diese besonders bevorzugten Eigenschaften besitzen insbesondere Copolylactide, die aus ε- Caprolacton als comonomere Einheit aufgebaut sind: Erfindungsgemäß sind aber auch überraschenderweise δ- Valerolacton, 1,4-Dioxanon-2 oder 1,3-Dioxanon-2 geeignet, wie auch Gemische aus den einzelnen Comonomeren, wodurch sich gegebenenfallls eine Feinabstimmung der gewünschten Eigenschaften erzielen läßt.

Wie bei nahezu jeder polymeren Verbindung sind die Eigenschaften der erfindungsgemäßen Copolylactide von den Verfahrensbedingungen und - parametem ihrer Herstellung abhängig.

Gegenstand der Erfindung ist somit auch ein Verfahren zur Herstellung eines entsprechenden Copolylactids, worin ein racemisches Lactid mit einem Comonomer, ausgewählt aus ε- Caprolacton, δ- Valerolacton, 1,4-Dioxanon-2 oder 1,3-Dioxanon-2 oder deren Gemischen umgesetzt wird. Dabei beträgt das Molverhältnis von Lactid zu Comonomer 90 - 80 : 10-20. Besonders bevorzugt sind die Lactid/Comonomer-Verhältnisse 84 : 16, 85 : 15 oder 86 : 14.

Entscheidend für die Polymerisation der monomeren Einheiten zu den Coplymeren mit den geforderten Eigenschaften ist der Einsatz von Zinn(ll)-diethylhexanoat als Initiator und eines Cokatalysators zur exakten Steuerung des Polymerisationsprozesses.

Um besonders gute Ergebnisse zu erzielen, ist es wichtig, daß der Initiator unmittelbar vor der Polymerisationsreaktion gereinigt wird. Da der käufliche Initiator (Zinn(II)-(diethylhexanoat)) bis zu 20 % Verunreinigungen durch Wasser und freier Octansäure enthalten kann, sollten diese entfernt werden. Der flüssige Initiator kann beispielsweise mit Xylol (preiswert, nicht toxisch) oder alternativ mit Toluol versetzt und ein- oder mehrfach als Azetrop abdestilliert werden. Anschließend empfiehlt sich eine fraktionierte Destillation im Hochvakuum (etwa 10⁻³ mbar). Andere Aufreinigungsverfahren, welche zu einer weitgehenden Entfernung der genannten Verunreinigungen führen, sind ebenfalls möglich.

Das Molverhältnis von Initiator zu Lactid/Comonomer, welches in dem erfindungsgemäßen Verfahren eingesetzt wird, beträgt 1 : 30.000 bis 1 : 50.000; vorzugsweise kommt ein Verhältnis von etwa 1: 40.000 (± 5%) zum Einsatz. Eine Erhöhung dieses Verhältnisses bewirkt eine Erhöhung des-Anteils an längeren Molekülketten und dadurch auch eine unerwünschte Erhöhung des Erweichungspunktes. Die Lehre der EP 0509 203 offenbart hingegen ein Verhältnis Initiator zu Lactid/Monomer von 1 : 100 bis 1 : 500. Dies hat nicht nur Auswirkungen auf die Beschaffenheit des Copolymers als solches, sondern bringt auch den Vorteil mit sich, daß die Menge an toxischem Zinn-Initiator etwa um den Faktor 1000 (bezogen auf das Molverhältnis) reduziert werden kann.

Erfindungswesentlich ist auch der Einsatz von Cokatalysatoren zur exakten Steuerung der Kettenlängen. Als geeignete Cokatalysatoren sind vor allem n-Alkanole mit vorzugsweise bis zu 18 C-Atomen aber auch Oligoethylenglykolmonomethylether oder Polyole oder Gemische von diesen zu nennen. Besonders bevorzugt ist n-Butanol.

Auch bei dem Einsatz des Cokatalysators ist auf die Einhaltung der Molverhältnisse zu achten. So liegt erfindungsgemäß das Molverhältnis Cokatalysator zu Lactid/Comonomer im Bereich von 1 : 200 bis 1 : 600, vorzugsweise im Bereich von 1 : 300 bis 1 : 500. Es hat sich herausgestellt, daß der so verwendete Cokatalysator eine wesentliche Rolle bei der Molekulargewichtsverteilung spielt. Somit erübrigt sich der aus der EP 0509 203 bekannte Versuch, kurzkettige Copolymere durch deren Auswaschen mit Alkohol in größeren Mengen aus dem polymeren Endprodukt zu entfernen.

Falls erwünscht, können dem Reaktionsgemisch Weichmacher zugesetzt werden, um die Glasübergangstemperatur und die Dampfdurchlässigkeit zu verändern. Als Weichmacher werden z.B. Glycerin, Phthalsäureester, Tributylcitrat oder überschüssiges Caprolacton verwendet. Der Anteil an Weichmachern sollte üblicherweise 10 bis 20 Gew. % nicht überschreiten.

Die Polymerisation von racemischem Lactid und der comonomeren Verbindung(en) erfolgt bei einer Temperatur von maximal 165°C. In einer Verfahrensvariante wird in einem Temperaturschritt bei einer Temperatur zwischen 155 und 165°C, vorzugsweise etwa 160°C in einem Zeitraum von 40 bis 55 h, vorzugsweise 46 bis 50 h, insbesondere etwa 48 h polymerisiert. Eine bevorzugte Verfahrensvariante benutzt einen zweistufigen Polymerisationsschritt: So wird zunächst bei einer Temperatur von 155 bis 165°C, vorzugsweise etwa 160°C, in einem Zeitraum von 20 bis 28 h, vorzugsweise etwa 24 h, und anschließend bei einer Temperatur von 90 bis 120 °C, vorzugsweise etwa 100°C, in einem Zeitraum von weiteren 20 bis 28 h, vorzugsweise etwa 24 h, polymerisiert. Die Temperaturwahl und - abfolge sowie die Dauer der Polymerisation ist letztlich abhängig von den gewünschten Eigenschaften des Polymerisats.

Nach abgeschlossener Polymerisation wird das Produkt in einer Ausführungsvariante einem Evakuierungsprozeß unterzogen, der dazu dient, flüchtige Substanzen aus der Polymerisationsmischung zu entfernen. Je nach Vakuum dauert dieser Vorgang erfindungsgemäß 10 bis 80, vorzugsweise 40 bis 60 Minuten. Das Vakuum wird bei Temperaturen zwischen Raumtemperatur und vorzugsweise 50 bis 120°C, insbesondere 100°C angelegt.

Wie bereits oben erwähnt, ist es wichtig, festzustellen, daß es mittels des erfindungsgemäßen Verfahrens gelingt, das Molekulargewicht des Copolymers, insbesondere das Verhältnis M_{w} / Mₙ, welches letztlich mit entscheidend für die geforderten Eigenschaften ist, in exakterer Weise einzustellen bzw. zu regeln, wie dies bislang nach dem Stand der Technik für derartige Produkte nicht möglich war. Somit ist Gegenstand der vorliegenden Erfindung auch ein Verfahren zur Steuerung des Verhältnisses M_{w} / Mₙ in einem Bereich von 1,2 bis 2,0, vorzugsweise 1,4 bis 1,7 bei der Herstellung eines Copolylactids, welches aus polymerisierten Einheiten von racemischen Lactid und den Comonomeren ε- Caprolacton, δ- Valerolacton, 1,4-Dioxanon-2 oder 1,3-Dioxanon-2 aufgebaut ist, worin die oben und in den Ansprüchen angegebenen Verfahrensschritte zur Anwendung kommen.
Die erfindungsgemäßen Copolylactide lassen sich nach Erkaltung oder noch als warmes Polymerisat ohne weiteren Zusatz zu Folien (Dicke 10 bis 100 µm) auswalzen und können so für vielfältige medizinische und kosmetische Zwecke eingesetzt werden. Haupteinsatzgebiet ist die Verwendung als chirurgische Operationsinzisionsfolie, insbesondere zur Wundabdekkung.

Alternativ können die erfindungsgemäßen Copolylactide auch in geeigneten Lösungsmitteln wie beispielsweise Essigsäureethylester, Aceton oder Methylenchlorid gelöst werden und mit bekannten Desinfektionsmitteln und/oder mit lokal wirkenden Anästhetika versetzt werden. Vorzugsweise wird Essigsäureethylester als Lösungsmittel eingesetzt, da es bereits über eine desinfizierende Wirkung verfügt.

Weiterhin können die Copolylactide besonders günstig als flüssiger Handschuh beim Umgang mit Allergenen eingesetzt werden. So sind sie beispielsweise auch für Allergiker bei Spül- oder Waschmittelallergien eine echte Alternative.

Bei der Verwendung als Sonnenschutzmittel werden der fertigen Lösung die üblichen UVA- und UVB-Filter beigemischt.

Die erfindungsgemäßen Copolylactide können auch als resorbierbarer Klebstoff eingesetzt werden, wenn sie in hierfür geeigneten Lösungsmitteln (z. B. Essigsäureethylester) gelöst werden.

Um zu zeigen, daß die Copolymere des Standes der Technik, insbesondere der Lehren der US 4,045,418 und der EP 0509 203, andere Eigenschaften besitzen und somit sich substantiell von denen der Erfindung unterscheiden, wurden erfindungsgemäß in Vergleichsversuchen nach den dort angegebenen Verfahren zu ihrer Herstellung vorgegangen und die erhaltenen Produkte mit denen der vorliegenden Erfindung verglichen. Hierzu wurden in allen Versuchen die gleichen Parameter bzw. Eigenschaften gemessen, womit ein direkter Vergleich erst ermöglicht wurde.

In der folgenden Tabelle werden die Eigenschaften eines Copolymers gemäß der Erfindung (Beispiel 2) mit einem Copolymer gemäß der US 4,045,418 (dortiges Beispiel 3) und einem Copolymer gemäß der EP-509 203 (dortiges Beispiel 1) dargestellt.

| **Meßparameter** | **Erfindung** | **US 4,045,418** | **EP 0509 203** |
|---|---|---|---|
| Lactid/ ε-Caprolacton | 85/15 | 85/15 | 85/15 |
| T_{g} (°C) | 37 | 49 | 22 |
| Viskosität ηᵢₙₕ (dl/g) | 0.6 | 1.6 | 0.31 |
| Molekulargew. M_{w} | 48000 | 214.300 | 38300 |
| Molekulargew. Mₙ | 30000 | 34600 | 17410 |
| M_{w}/Mₙ-Quotient | 1.6 | 6.2 | 2.2 |
| Verhalten als Folie auf der Haut bei 37° | Gute Transparenz, langsame Hydrolyse, adhäsiv Flexibel, anschmiegsam | Wenig flexibel, steif brüchig, schlechte Adhäsion | Klebrig, mäßige Transparenz, rasche Trübung der Folie |
| Natürlicher, hydrolytischer Abbau der Sprühfolie | 4- 6 Wo | nicht meßbar | bis zu 30 Wo |

Die Tabelle zeigt deutlich, daß trotz gleichem Lactid/Comonomer-Verhältnis die drei Copolylactide unterschiedliche Eigenschaften besitzen und sich somit unterscheiden. Lediglich das erfindungsgemäße Copolylactid mit einer engen Molekulargewichtsverteilung von unter 2,0 (hier 1.6), erfüllt das gewünschte Anforderungsprofil hinsichtlich Flexibilität, Transparenz, Adhäsion und Anschmiegsamkeit an die Haut.
Das Copolymer gemäß EP 0509 203 besitzt aufgrund der niedrigen Glasübergangstemperatur von 22 °C und der niedrigen Viskosität eine klebrige Konsistenz, so daß dies zu Verklebungen mit aufliegenden Textilien (z.B. Verbandsmaterial) führen muß.
Die Viskosität der erfindungsgemäßen Copolymere liegt in der Regel zwischen 0,30 und 0,75, vorzugsweise zwischen 0,55 und 0,67. Damit unterscheiden sie sich auch von denen der beiden genannten Patentanmeldungen, die entweder höhere oder niedrigere Viskosität aufweisen.

Anhand der folgenden Beispiele wird die Erfindung näher erläutert:

Alle Charakterisierungsmethoden wurden wie folgt durchgeführt: DSC- Messungen in Al-Pfännchen unter Stickstoff mit einer Heizrate von 20 °C/min und die T_{g}-Werte der 1. Aufheizkurve gelistet.
Inhärente Viskositäten bei 25 °C mit einer Polymerkonzentration c = 2 g/l in Dichlormethan mit automatisiertem Ubbelohde-Viskosimeter (Fa. Lauda). GPC- Messungen in THF bei 30 °C mit Poly(ε-Caprolacton)-Eichung.

Zur Reinigung des flüssigen Initiators wurde mit Xylol (preiswert, nicht toxisch) oder alternativ mit Toluol versetzt und zweimal als Azetrop abdestilliert. Anschließend wurde im Hochvakuum (10⁻³ mbar) fraktioniert destilliert, wobei die höchstsiedende Fraktion als Endprodukt verwendet wurde.
Alle beschriebenen Versuche wurden ausschließlich mit gereinigtem Initiator durchgeführt. Dies gilt auch für die Vergleichsversuche des Standes der Technik.

### Beispiel 1: (Lactid/ε-Caprolacton 85:15, n-Butanol 200 : 1)

24.5 g (170 mmol) D,L-Lactid (Boehringer S-Grade) und 3.42 g (30 mmol) ε-Caprolacton (über CaH₂ destilliert) wurden in einem 100 ml Rundkolben eingewogen, und die Lösung von 2 mg (0.005 mmol) SnOct₂ (Aldrich, vorher gereinigt) sowie 74 mg (1 mmol) n-Butanol in 2 ml trockenem Diethylether wurden dazu pipettiert. Der mit einem Glasstopfen verschlossene Kolben wurde in ein auf 160 °C geheiztes Ölbad gebracht, durch kurzes Öffnen der Überdruck abgelassen und durch Rühren mit einem Magnetrührer eine Durchmischung der Reaktionspartner erreicht. Nach 24 h wurde die Temperatur auf 100 °C gesenkt und nach weiteren 24 h der Versuch abgekühlt. Danach wurde noch 1 h bei 100 °C evakuiert. Für das glasartige Rohprodukt wurde eine Glastemperatur T_{g} = 31 °C gemessen, eine inhärente Viskosität ηᵢₙₕ= 0.40 dl/g, ein Molekulargewicht Mₙ = 16000 sowie M_{w} = 24000. Daraus ergibt sich ein M_{w}/ Mₙ-Quotient von 1.5.

### Beispiel 2: (Lactid/ε-Caprolacton 85:15, n-Butanol 400 : 1)

24.5 g (170 mmol) D,L-Lactid (Boehringer S-Grade) und 3.42 g (30 mmol) ε-Caprolacton (über CaH₂ destilliert) wurden in einem 100 mll Rundkolben eingewogen, und die Lösung von 2 mg (0.005 mmol) SnOct₂ sowie 37 mg (0.5 mmol) n-Butanol in 2 ml trockenem Diethylether wurden dazu pipettiert. Das Reaktionsgemisch wurde 24 h bei 160 °C und danach 24 h bei 100 °C polymerisiert. Für das anschließende evakuierte Rohprodukt wurden folgende Werte ermittelt: T_{g} = 38 °C; ηᵢₙₕ= 0.60 dl/g; Mₙ = 30000 und M_{w} = 48000; M_{w}/ Mₙ= 1.6.

### Beispiel 3: (Lactid/1,4-Dioxanon-2 85:15, Triethylenglycolmonomethylether 200 : 1)

24.5 g (170 mmol) D,L-Lactid und 3.06 g ( 30 mmol) 1.4-Dioxanon-2 (frisch dest.) wurden in einen 100 ml Rundkolben eingewogen, und die Lösung von 2 mg (0.005 mmol) SnOct₂ und 164 mg ( 1 mmol) Triethylenglycolmonomethylether in 2 ml tr. Diethylether zugegeben. Das Reaktionsgemisch wurde wie in Beispiel 1 polymerisiert. Für das evakuierte Rohprodukt wurden folgende Eigenschaften gemessen:
T_{g} = 37 °C; ηᵢₙₕ= 0.35 dl/g; Mₙ = 15000 und M_{w} = 21900; M_{w}/ Mₙ= 1.46

### Beispiel 4: (Lactid/1,3-Dioxanon-2 85:15, n-Butanol 400 : 1)

24.5 g (170 mmol) D,L-Lactid und 3.06 g ( 30 mmol) 1.3-Dioxanon-2 (Trimethylencarbonat, Boehringer S-Grade) wurden in einen 100 ml Rundkolben eingewogen, und die Lösung von 2 mg (0.005 mmol) SnOct₂ und 37 mg ( 0.5 mmol) n-Butanol in 2 ml trockenem Diethylether zugegeben. Das Reaktionsgemisch wurde wie in Beispiel 1 polymerisiert. Für das evakuierte Rohprodukt wurden folgende Eigenschaften gemessen: T_{g} = 40°C; ηᵢₙₕ= 0.68 dl/g; Mₙ = 38000 und M_{w} = 55000; M_{w}/ Mₙ= 1.4.

### Beispiel 5 (Lactid/ε-Caprolacton 85:15, n-Butanol 200 : 1):

Analog Beispiel 1 wird ein Copolylactid hergestellt mit der Ausnahme, daß die Polymerisation in einem einzigen Temperaturschritt bei 160°C in einem Zeitraum von 36 h durchgeführt wurde. Für das glasartige Rohprodukt wurde eine Glasübergangstemperatur T_{g} = 30 °C gemessen, eine inhärente Viskosität ηᵢₙₕ= 0.38 dl/g, ein Molekulargewicht Mₙ = 18000 sowie M_{w} = 33000. Daraus ergibt sich ein M_{w}/Mₙ-Quotient von 1.8.

## Patentansprüche

1. Flexibles, transparentes Copolylactid, welches aus polymerisierten Einheiten von racemischen Lactid und den Comonomeren ε- Caprolacton, δ- Valerolacton, 1,4-Dioxanon-2 oder 1,3-Dioxanon-2 aufgebaut ist, eine Glasübergangstemperatur zwischen 30 und 43°C, ein Zahlenmittel des Molekulargewichts Mₙ von 15.000 bis 50.000 und eine Polydispersität Pₙ von 1,2 bis 2,0 aufweist.

2. Copolylactid nach Anspruch 1, **dadurch gekennzeichnet, daß** als comonomere Einheit ε-Caprolacton vorliegt.

3. Copolylactid nach der Anspruch 1 oder 2, **dadurch gekennzeichnet, daß** die Glasübergangstemperatur des Copolylactids zwischen 33 und 37°C liegt.

4. Copolylactid nach einem der Ansprüche 1 bis 3, **dadurch gekennzeichnet, daß** das Zahlenmittel des Molekulargewichts Mₙ = 25.000 bis 40.000 beträgt.

5. Copolylactid nach einem der Ansprüche 1 bis 4, **dadurch gekennzeichnet, daß** die Polydispersität Pₙ zwischen 1,4 und 1,7 liegt.

6. Verfahren zur Herstellung eines Copolylactids, **dadurch gekennzeichnet, daß** man racemischen Lactid mit einem Comonomer, ausgewählt aus ε- Caprolacton, δ- Valerolacton, 1,4-Dioxanon-2 oder 1,3-Dioxanon-2, wobei das Molverhältnis von Lactid zu Comonomer 90 - 80 : 10-20 beträgt, in Gegenwart von frisch destilliertem Zinn(II)- diethylhexanoat als Initiator, wobei das Molverhältnis von Initiator zu Lactid/Comonomer 1 : 30.000 bis 1 : 50.000 beträgt, sowie in Gegenwart von n-Alkanolen , Oligoethylenglykolmonomethylether oder Polyolen als Cokatalysator, wobei das Molverhältnis Cokatalysator zu Lactid/Comonomer im Bereich von 1 : 200 bis 1 : 600 liegt, bei einer Temperatur von maximal 165°C polymerisiert.

7. Verfahren nach Anspruch 6, **dadurch gekennzeichnet, daß** die Polymerisation bei einer Temperatur von 155 bis 165°C in einem Zeitraum von 20 bis 28 h und anschließend bei einer Temperatur von 90 bis 120 °C in einem Zeitraum von weiteren 20 bis 28 h durchgeführt wird.

8. Verfahren nach Anspruch 6 oder 7, **dadurch gekennzeichnet, daß** das Molverhältnis Lactid zu Comonomer 84 : 16, 85 : 15 oder 86 : 14 beträgt.

9. Verfahren nach einen der Ansprüche 6 bis 8, **dadurch gekennzeichnet, daß** das Molverhältnis Initiator zu Lactid/Comonomer etwa 1 : 40.000 beträgt und das Molverhältnis Cokatalysator zu Lactid/Comonomer zwischen 1 : 300 bis 1: 500 liegt.

10. Verfahren nach einem der Ansprüche 6 bis 9, **dadurch gekennzeichnet, daß** man das polymerisierte Produkt einer Vakuumbehandlungunterzieht.

11. Flexibles, transparentes Copolylactid erhältlich nach einem Verfahren gemäß der Ansprüche 6 bis 10.

12. Copolylactid nach einem der Ansprüche 1 bis 5 oder Anspruch 11, **dadurch gekennzeichnet, daß** es zusätzlich einen Weichmacher enthält.

13. Verfahren zur Steuerung der Molekulargewichtsverteilung M_{w} / Mₙ in einem Bereich von 1,4 bis 2,0 bei der Herstellung eines Copolymers, welches aus polymerisierten Einheiten von racemischen Lactid und den Comonomeren ε- Caprolacton, δ- Valerolacton, 1,4-Dioxanon-2 oder 1,3-Dioxanon-2 aufgebaut ist, **dadurch gekennzeichnet, daß** man die Verfahrensschritte gemäß der Ansprüche 6 bis 10 ausführt.

14. Zusammensetzung, enthaltend ein Copolylactid gemäß Ansprüche 1 bis 5 oder Anspruch 11 zusammen mit einem Lösungsmittel, in welches das Copolylactid eingebracht worden ist.

15. Zusammensetzung nach Anspruch 14, enthaltend ein Desinfektionsmittel und/oder Anästhetikum.

16. Verwendung des Copolylactids nach einem der Ansprüche 1 bis 5 bzw. 11 zur Herstellung chirurgischer Operationsfolien, Wundabdekkungen oder flüssigen Handschuhe.

17. Verwendung des Copolylactids nach einem der Ansprüche 1 bis 5 bzw. 11 als Sonnenschutzmittel.

18. Verwendung des Copolylactids nach einem der Ansprüche 1 bis 5 bzw. 11 als resorbierbarer Klebstoff.

## Claims

1. Flexible, transparent copolylactide which is built up from polymerised units of racemic lactide and the comonomers ε-caprolactone, δ-valero-lactone, 1,4-dioxan-2-one or 1,3-dioxan-2-one, and has a glass transition temperature of between 30 and 43°C, a number average molecular weight Mₙ of 15,000 to 50,000 and a polydispersity Pₙ of 1.2 to 2.0.

2. Copolylactide according to Claim 1, **characterised in that** the comonomeric unit present is ε-caprolactone.

3. Copolylactide according to Claim 1 or 2, **characterised in that** the glass transition temperature of the copolylactide is between 33 and 37°C.

4. Copolylactide according to one of Claims 1 to 3, **characterised in that** the number average molecular weight Mₙ = 25,000 to 40,000.

5. Copolylactide according to one of Claims 1 to 4, **characterised in that** the polydispersity Pₙ is between 1.4 and 1.7.

6. Process for the preparation of a copolylactide, **characterised in that** racemic lactide is polymerised with a comonomer selected from ε-caprolactone, δ-valerolactone, 1,4-dioxan-2-one or 1,3-dioxan-2-one, where the molar ratio of lactide to comonomer is 90-80:10-20, in the presence of freshly distilled tin(II) diethylhexanoate as initiator, where the molar ratio of initiator to lactide/comonomer is 1:30,000 to 1:50,000, and in the presence of n-alkanols, oligoethylene glycol monomethyl ether or polyols as cocatalyst, where the cocatalyst to lactide/comonomer molar ratio is in the range from 1:200 to 1:600, at a maximum temperature of 165°C.

7. Process according to Claim 6, **characterised in that** the polymerisation is carried out at a temperature of 155 to 165°C in a period of 20 to 28 h and subsequently at a temperature of 90 to 120°C in a period of a further 20 to 28 h.

8. Process according to Claim 6 or 7, **characterised in that** the lactide to comonomer molar ratio is 84:16, 85:15 or 86:14.

9. Process according to one of Claims 6 to 8, **characterised in that** the initiator to lactide/comonomer molar ratio is about 1:40,000 and the cocatalyst to lactide/comonomer molar ratio is between 1:300 and 1:500.

10. Process according to one of Claims 6 to 9, **characterised in that** the polymerised product is subjected to vacuum treatment.

11. Flexible, transparent copolylactide obtainable by a process according to Claims 6 to 10.

12. Copolylactide according to one of Claims 1 to 5 or Claim 11, **characterised in that** it additionally comprises a plasticiser.

13. Process for controlling the molecular weight distribution M_{w}/Mₙ in a range of 1.4 to 2.0 in the preparation of a copolymer which is built up from polymerised units of racemic lactide and the comonomers ε-caprolactone, δ-valerolactone, 1,4-dioxan-2-one or 1,3-dioxan-2-one, **characterised in that** the process steps according to Claims 6 to 10 are carried out.

14. Composition comprising a copolylactide according to Claims 1 to 5 or Claim 11 together with a solvent into which the copolylactide has been introduced.

15. Composition according to Claim 14, comprising a disinfectant and/or anaesthetic.

16. Use of the copolylactide according to one of Claims 1 to 5 or 11 for the preparation of surgical operation films, wound covers or liquid gloves.

17. Use of the copolylactide according to one of Claims 1 to 5 or 11 as sunscreen.

18. Use of the copolylactide according to one of Claims 1 to 5 or 11 as absorbable adhesive.

## Revendications

1. Copolylactide transparent et flexible qui est élaboré à partir d'unités polymérisées de lactide racémique et de comonomères ε-caprolactone, δ-valérolactone, 1,4-dioxane-2-one ou 1,3-dioxane-2-one, et qui présente une température de transition vitreuse entre 30 et 43°C, un poids moléculaire moyen Mₙ de 15 000 à 50 000 et une polydispersité Pₙ de 1,2 à 2,0.

2. Copolylactide selon la revendication 1, **caractérisé en ce que** l'unité comonomérique présente est ε-caprolactone.

3. Copolylactide selon la revendication 1 ou 2, **caractérisé en ce que** la température de transition vitreuse du copolylactide est entre 33 et 37°C.

4. Copolylactide selon l'une des revendications 1 à 3, **caractérisé en ce que** le poids moléculaire moyen Mₙ = 25 000 à 40 000.

5. Copolylactide selon l'une des revendications 1 à 4, **caractérisé en ce que** la polydispersité Pₙ est entre 1,4 et 1,7.

6. Procédé pour la préparation d'un copolylactide, **caractérisé en ce qu'**un lactide racémique est polymérisé avec un comonomère choisi parmi ε-caprolactone, δ-valérolactone, 1,4-dioxane-2-one ou 1,3-dioxane-2-one, où le rapport molaire lactide sur comonomère est 90-80:10-20, en présence de diéthylhexanoate d'étain(II) fraîchement distillé en tant qu'initiateur, où le rapport molaire initiateur sur lactide/comonomère est 1:30 000 à 1:50 000, et en présence de n-alkanols, d'éther oligoéthylène glycol monométhyle ou de polyols en tant que cocatalyseur, où le rapport molaire cocatalyseur sur lactide/comonomère est dans la plage de 1:200 à 1:600, à une température maximum de 165°C.

7. Procédé selon la revendication 6, **caractérisé en ce que** la polymérisation est mise en oeuvre à une température de 155 à 165°C dans une période de 20 à 28 h et ensuite à une température de 90 à 120°C dans une période supplémentaire de 20 à 28 h.

8. Procédé selon la revendication 6 ou 7, **caractérisé en ce que** le rapport molaire lactide sur comonomère est 84:16, 85:15 ou 86:14.

9. Procédé selon l'une des revendications 6 à 8, **caractérisé en ce que** le rapport molaire initiateur sur lactide/comonomère est d'environ 1:40 000 et le rapport molaire cocatalyseur sur lactide/comonomère est entre 1:300 et 1:500.

10. Procédé selon l'une des revendications 6 à 9, **caractérisé en ce que** le produit polymérisé est soumis à un traitement sous vide.

11. Copolylactide transparent et flexible pouvant être obtenu au moyen d'un procédé selon les revendications 6 à 10.

12. Copolylactide selon l'une des revendications 1 à 5 ou la revendication 11, **caractérisé en ce qu'**il comprend de façon additionnelle un plastifiant.

13. Procédé pour contrôler la distribution de poids moléculaires M_{w}/Mₙ dans une plage de 1,4 à 2,0 au niveau de la préparation d'un copolymère qui est élaboré à partir d'unités polymérisées de lactide racémique et de comonomères ε-caprolactone, δ-valérolactone, 1,4-dioxane-2-one ou 1,3-dioxane-2-one, **caractérisé en ce que** les étapes de procédé selon les revendications 6 à 10 sont mises en oeuvre.

14. Composition comprenant un copolylactide selon les revendications 1 à 5 ou la revendication 11 en association avec un solvant dans lequel le copolylactide a été introduit.

15. Composition selon la revendication 14, comprenant un désinfectant et/ou un anesthésiant.

16. Utilisation du copolylactide selon l'une des revendications 1 à 5 ou 11 pour la préparation de films pour opérations chirurgicales, de recouvrements enroulés ou de gants liquides.

17. Utilisation de copolylactide selon l'une des revendications 1 à 5 ou 11 en tant qu'agent de protection solaire.

18. Utilisation du copolylactide selon l'une des revendications 1 à 5 ou 11 en tant qu'adhésif résorbable.
